# EUROPEAN PATENT SPECIFICATION

(11) **EP 3 061 753 B1**
(45) Date of publication and mention of the grant of the patent: **22.08.2018**
(21) Application number: 16156888.6
(22) Date of filing: 23.02.2016
(51) Int. Cl.: C07D 239/22, C07C 227/22, C07C 229/36, C07K 7/00

(54) **ENANTIO-SELECTIVE SYNTHESIS OF NON-NATURAL AMINO ACIDS**
ENANTIOSELEKTIVE SYNTHESE VON NICHTNATÜRLICHEN AMINOSÄUREN
SYNTHÈSE ÉNANTIOSÉLECTIVE D'ACIDES AMINÉS NON NATURELS

(30) Priority: 27.02.2015 IT RM20150091
(43) Date of publication of application: 31.08.2016
(73) Proprietor: Universita' Degli Studi "G. d'Annunzio" Chieti-Pescara, 66013 Chieti (IT)
(72) Inventor: MOLLICA, Adriano, 66100 CHIETI (IT); COSTANTE, Roberto, 66100 CHIETI (IT)
(74) Representative: Currado, Luisa

(56) References cited:
- TINGYOU LI: "Enantioselective synthesis of phenylalanine library containing alkyl groups on the aromoatic moiety: confirmation of stereostructure by X-Ray analysis", CHEMICAL AND PHARMACEUTICAL BULLETIN, 1 June 2006 (2006-06-01), pages 873-877, XP055223368,
- HARUHIKO AOYAGI ET AL: "Synthesis of unusual aromatic L-amino acids by asymmetric hydrogenation of cyclic dehydrodipeptides.", BULLETIN OF THE CHEMICAL SOCIETY OF JAPAN, vol. 59, no. 1, 1 January 1986 (1986-01-01), pages 323-324, XP055222917, JP ISSN: 0009-2673, DOI: 10.1246/bcsj.59.323
- TANG X ET AL: "Convenient, asymmetric synthesis of enantiomerically pure 2',6'-dimethyltyrosine (DMT) via alkylation of chiral equivalent of nucleophilic glycine", TETRAHEDRON ASYMMETRY, PERGAMON PRESS LTD, OXFORD, GB, vol. 11, no. 14, 28 July 2000 (2000-07-28) , pages 2917-2925, XP004214065, ISSN: 0957-4166, DOI: 10.1016/S0957-4166(00)00250-0

## Description

### Field of the invention

The present invention refers to the chemical field, in particular to organic chemistry and more in particular to the preparation of unnatural aromatic amino acids with a process allowing the synthesis of specific enantiomers by using non-chiral catalyst.

### Background of the invention

Unnatural amino acids are usually introduced in peptides with biological activity to obtain peptides and peptidomimetics showing new biological features.

Of particular interest are phenylalanine and tyrosine di-methylated in positions 2' and 6' of the aromatic ring (also known as DMP e DMT respectively) with restricted structure, being enantiomerically pure isomer D or L, having conformational rigidity of the side chains which cannot rotate because of the presence of methyl groups in the two ortho positions.

Said unnatural amino acids are used to substitute the corresponding natural amino acids when investigating the binding of peptidic ligands, and also for designing biologically active peptidomimetics with induced affinity and/or selectivity for certain receptors (Koda, Y. et al. Bioorg. Med. Chem., 2008, 16, 6286; Balboni, G. et al. Bioorg. Med. Chem., 2010, 18, 6024; Fichna, J. et al. J. Med. Chem. 2007, 50, 512).

For example, 2',6' di-methyl tyrosine (DMT) and 2',6' di-methyl phenylalanine (DMP) can influence the activity of opioid peptides, such as endorphin 2, increasing the affinity for µ and δ receptors (Li, T. et al. J. Med. Chem., 2007, 50, 2753-2766).

The synthesis of DMT and DMP by stereo-selective asymmetric hydrogenation of α-β dehydro-tyrosine, carrying a protective group in the presence of [Rh(1.5-COD)(R,R-DIPAMP)]BF₄ chiral catalysts, is known in the art (Dygos, J.H. et al. Synthesis 1992, 8, 741; Li, T. et al. Chem. Pharm. Bull. 2006, 54, 873).

It is also know the synthesis of DMT based on the stereo-selective alkylation of di-cheto-piperazine in the presence of a bulky chiral group on the nitrogen atoms, even if this process avoid the use of chiral catalysts it needs several steps for synthetizing the intermediates of the reaction (Balducci, D. et al. Tetrahedron Asymm. 2009, 20, 1398).

WO2004055195 discloses an enzymatic separation of R or S enantiomers of DMP in a racemic mixture.

Recently, the inventors of the present application have prepared 2,5-dichetopiperazine by condensation of dipeptide-esters having protective groups at N-terminal, which is a key intermediate of the process object of the present invention (Mollica A et al., Fitoterapia, 2014,98,91-97).

It is clear that the processes, known in the art, for synthetizing single enantiomers of aromatic unnatural amino acids are based on the separation of single enantiomers in racemic mixtures or involve chiral catalysts or the presence of bulky chiral groups.

On the base of what was known in the art and with the aim of providing a simple process, using easily available reactants, and avoiding the use of expensive chiral catalysts, the same inventors developed a process for the enantio-selective synthesis of aromatic unnatural amino acids, with restricted structure being enantiomerically pure isomer D or L, using easy available and cheap diketopiperazines, derived from condensation of simple dipeptides, as key intermediates, which undergo to aldol condensation with easy available and cheap aromatic aldehydes in the presence of a non-chiral catalyst.

### Object of the invention

The above technical problem is solved by providing a process for the stereo-selective preparation of L stereoisomers of unnatural aromatic amino acids optionally substituted on the aromatic ring of formula comprising the following steps:
a) coupling a natural amino acid having an alkyl or aromatic side chain, having S or R absolute configuration and having a protective group (GP) on the amine with a glycine C1-C4 alkyl ester, linear or branched, to obtain dipeptide of formula (I): 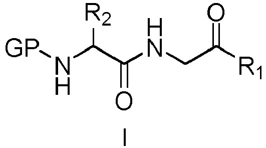
   wherein R1 is methoxy;
   R2 is isopropyl;
b) removing the protective group (GP) from the compound of formula (I) as prepared in step a) by reacting with a suitable reactive to obtain dipeptide of formula (II) or salt thereof: 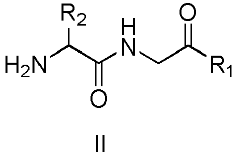 wherein R1 and R2 have the same meaning of step a);
c) intramolecular condensation of dipeptide of formula (II) as prepared in step b) to obtain compound of formula (III): 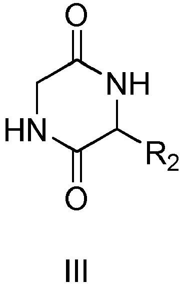 wherein R2 has the same meaning of the preceding steps;
d) acetylation of compound of formula (III) as prepared in step c) to obtain compound of formula (IV): 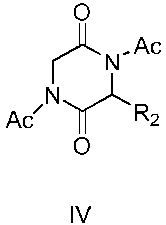 wherein Ac is acetyl and R2 has the same meaning of the preceding steps;
e) condensation of compound of formula (IV) as prepared in step d) with aldehyde of formula (V) to obtain Z-alkene of formula (VI): 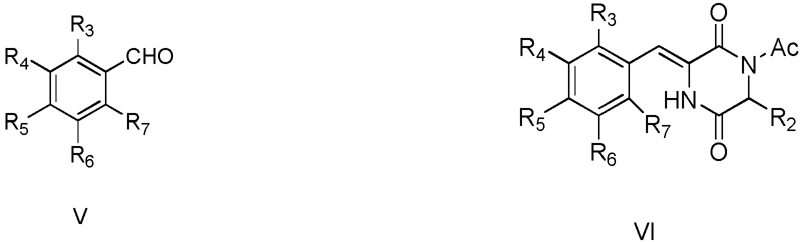
   wherein R3 and R7 are CH₃;
   R4 and, R6, are H; , R5 is H or benzyloxy;
   Ac is acetyl and R2 has the same meaning of the preceding steps;
f) removal of acetyl residue from compound of (VI) as prepared in step e) to obtain compound of formula (VII): 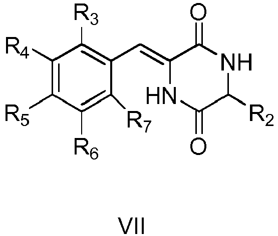 wherein R2, R3, R4, R5, R6 e R7 have the same meaning of the preceding step e);
g) catalytic hydrogenation of compound of formula (VII) as prepared in step f) in presence of a achiral catalyst, to obtain compound of formula (VIII): 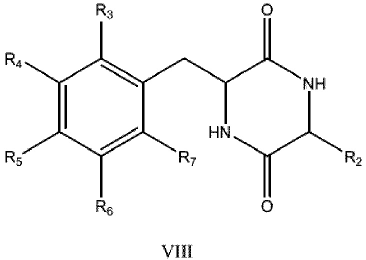 wherein R2, R3, R4, R6 e R7 have the same meaning of the preceding step and R5, is H or OH;
h) hydrolysis of compound of formula (VIII) as prepared in step g) to obtain unnatural aromatic amino acids optionally substituted on the aromatic ring of formula (IX) in mixture with an amino acid of formula (X): 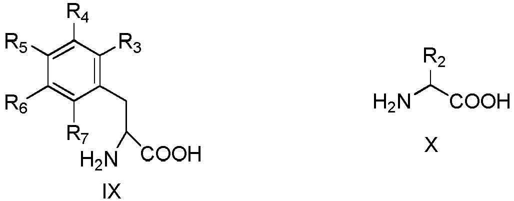
   Wherein compound of formula (IX) is 2',6'-dimethyl-L-Tyrosine, 2',6'-dimethyl-L-Phenylalanine
   R2 has the same meaning of the preceding steps;
   Optionally step h) is further followed by the following steps:
i) protecting unnatural aromatic amino acids optionally substituted on the aromatic ring of formula (IX) and amino acids of formula (X) as prepared in step h) with a protector group GP on N- terminus, to obtain compounds of formula (XI) and (XII) 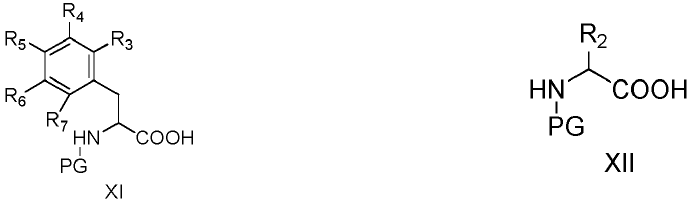
j) separation of compounds as prepared in step i) to obtain pure compound of formula (IX). Further characteristic of the present invention will be clarified by the following detailed description.

### Detailed description of the invention

The present invention provides a process for the stereo-selective preparation of L stereoisomers of unnatural aromatic amino acids optionally substituted on the aromatic ring.

The unnatural aromatic amino acids optionally substituted on the aromatic ring obtained by the process of the present invention have formula (IX):

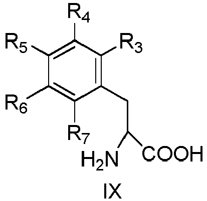

Wherein the unnatural aromatic amino acids of formula (IX) obtained with the process of the present invention are 2',6'-dimethyl-L-Tyrosine, 2',6'-dimethyl-L-Phenylalanine.

Said unnatural aromatic amino acids are prepared with the process of the present invention comprising the following steps:
a) coupling a natural amino acid having an alkyl or aromatic side chain, having S or R absolute configuration and having a protective group (GP) on the amine with a glycine C1-C4 alkyl ester, linear or branched, to obtain dipeptide of formula (I): 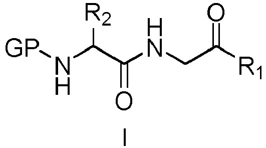
   wherein R1 is methoxy;
   R2 is isopropyl;
b) removing the protective group (GP) from the compound of formula (I) as prepared in step a) by reacting with a suitable reactive to obtain dipeptide of formula (II) or salt thereof: 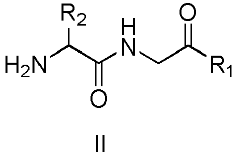 wherein R1 and R2 have the same meaning of step a)
c) intramolecular condensation of dipeptide of formula (II) as prepared in step b) to obtain compound of formula (III): 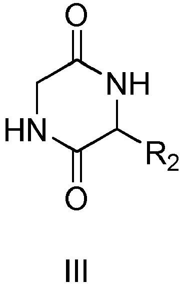 wherein R2 has the same meaning of the preceding steps;
d) acetylation of compound of formula (III) as prepared in step c) to obtain compound of formula (IV): 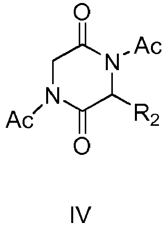 wherein Ac is acetyl and R2 has the same meaning of the preceding steps;
e) condensation of compound of formula (IV) as prepared in step d) with aldehyde of formula (V) to obtain Z-alkene of formula (VI): 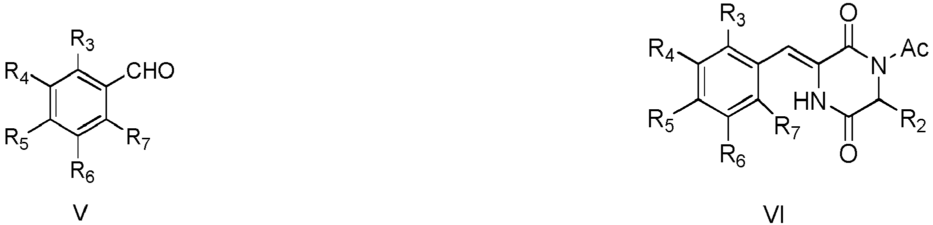
   wherein R3 and R7are CH₃;
   R4, ,R6, are H, R5 is H or benzyloxy;
   Ac is acetyl and R2 has the same meaning of the preceding steps;
f) removal of acetyl residue from compound of (VI) as prepared in step e) to obtain compound of formula (VII): 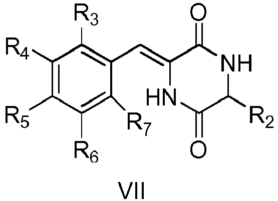 wherein R2, R3, R4, R5, R6 e R7 have the same meaning of the preceding step e);
g) catalytic hydrogenation of compound of formula (VII) as prepared in step f) in presence of a achiral catalyst, to obtain compound of formula (VIII): 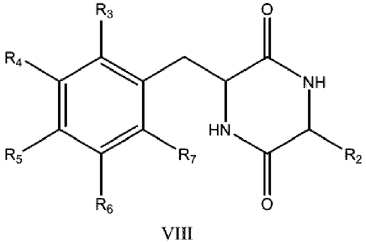 wherein R2, R3, R4, R6 e R7 have the same meaning of the preceding step and R5 is H or OH;
h) hydrolysis of compound of formula (VIII) as prepared in step g) to obtain unnatural aromatic amino acids optionally substituted on the aromatic ring of formula (IX) in mixture with an amino acid of formula (X): 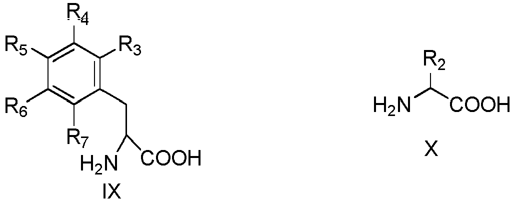 wherein R2, R3, R4, R6 e R7 have the same meaning of the preceding step; R5 is H or OH;
   Optionally step h) is further followed by the following steps:
i) protecting unnatural aromatic amino acids optionally substituted on the aromatic ring of formula (IX) and amino acids of formula (X) as prepared in step h) with a protector group GP on N- terminus, to obtain compounds of formula (XI) and (XII) 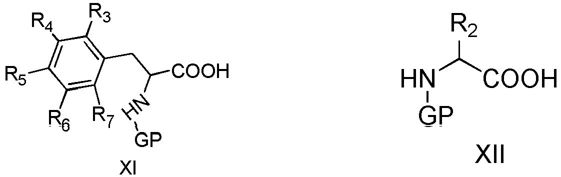
k) separation of compounds as prepared in step i) to obtain pure compound of formula (IX).

Preferably in step b) the salts of compound (I) are selected from the group consisting of: salts of hydrochloric acid (hydrochlorides), salts of sulfuric acid (sulfates), salts of trifluoroacetic acid (trifluoroacetates), acid salts bromide (hydrobromides). More preferably are salts of hydrochloric acid, trifluoroacetic acid salts.

Preferably in steps a) and i) the protective group (GP) is tert-butyloxycarbonyl protecting group (BOC). Preferably in step b) the suitable reagent for eliminating the protective group (GP) is selected from the group consisting of: pure Trifluoroacetic acid or Trifluroacetic acid in mixture with dichloromethane, hydrochloric acid in methanol or ethanol or THF or dioxane.

More preferably the suitable reagent used to eliminate the protective group (GP) is trifluoroacetic acid (TFA) in dichloromethane in ratio 1:1.

Preferably in step g) non-chiral catalyst is Palladium 10%, Palladium 5%.

More preferably in step g) non-chiral catalyst is Palladium 5%.

Preferably in step g) non-chiral catalyst is adsorbed on coal.

In a preferred embodiment in step g) the reaction is performed in a reactor.

In a preferred embodiment in step g) compound of formula (VII) is dissolved in methanol before adding the non-chiral catalyst.

In a preferred embodiment in step g) hydrogenation of compound (VII) last for at least 24 hours, at hydrogen pressure ranging between 2 and 10 atmosphere corresponding to 202650 and 1013250 Pa, preferably 607950-810600 Pa (6-8 atmospheres), and a temperature comprised between 45°C-60°C, preferably 45°C.

In a preferred embodiment in step g) at the end of hydrogenation reaction the mixture is filtered to remove the catalyst and the solvent evaporated to obtain the compound of formula (VIII).

The process for the preparation of 2',6'-dimethyl-L-Tyrosine (DMT) and 2',6'-dimethyl-L-Phenylalanine (DMF) comprises the following steps:
a) coupling amino acid L-valine, protected on the amino group with a protective group with glycine methyl ester, to obtain the dipeptide of formula (I): 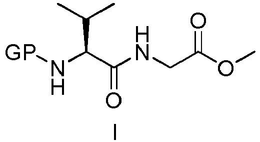
b) removal of the protective group GP by reacting with a suitable reactant to obtain the dipeptide methyl ester of formula (II): 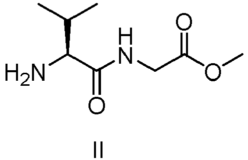
c) condensating the dipeptide obtained in step b) to obtain the compound of formula (III): 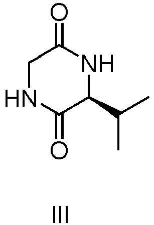
d) acetylation of the compound of formula (III) to obtain the compound of formula (IV): 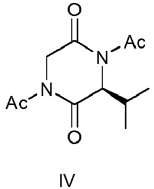
e) condensation of the compound of formula (IV) with an aldehyde of formula (V) to obtain a Z-alkene of formula (VI): 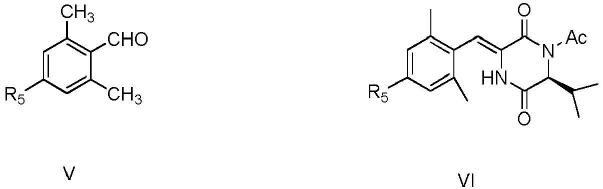 wherein R5 is H or benzyloxy
f) removal of the acetic group of compound of formula (VI) to give the compound (VII): 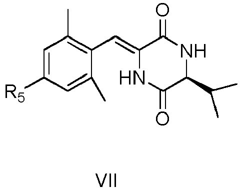 wherein R5 is H or benzyloxy
g) catalytic hydrogenation in the presence of a non-chiral catalyst in hydrogen atmosphere to obtain the compound of formula (VIII): 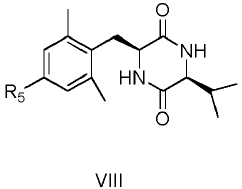 wherein R5 is H or OH
h) hydrolysis of compound of (VIII) to obtain 2',6'-dimethyl-L-Tyrosine (DMT) and 2',6'-dimethyl-L-Phenylalanine (DMF) in mixture with L-valine as a HCl salt. 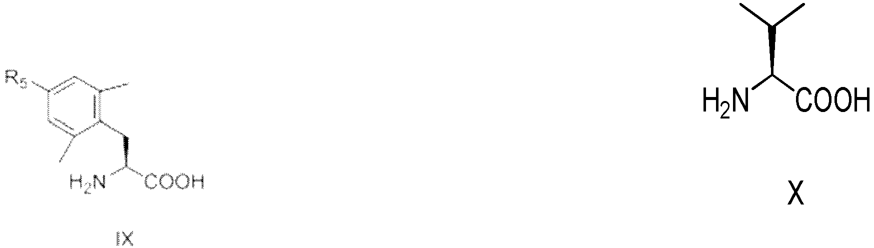 wherein R5 is H or OH
i) protection with a protective group GP on the N-terminal of compounds IX and X obtained in step h): 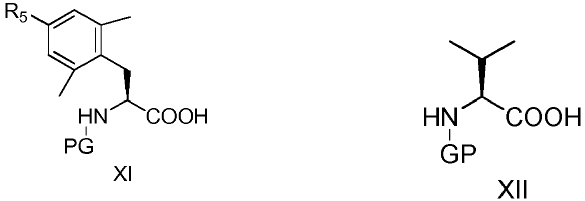 wherein R5 is H or OH
j) Separation of compounds obtained in step i) to obtain pure 2',6'-dimethyl-L-Tyrosine (DMT) and 2',6'-dimethyl-L-Phenylalanine (DMF).

### Examples

Nature of compounds was confirmed by ¹HNMR spectrum recorded with spectrometer Varian Inova 300 MHz (Varian Inc, Palo Alto, CA, USA). Chemical shifts were reported as parts per million (δ) in comparison with internal standard, tetramethylsilane (Me₄Si). Homogeneity confirmed with thin layer chromatography on silica gel Merck 60 F254 (Merck, Germany).

Solutions were usually dried on anhydrous Na₂SO₄ before evaporation. Chromatographic purifications were made on silica gel column Merck 60 with mesh 70-230. All the chemical products had the highest purity commercially available. 2-6-dimethylbenzaldehyde and 2,6-dimethy-4-hydroxybenzaldehyde purchased from Fluorochem, all the other reactants and solvents purchased from Sigma-Aldrich. All the linear dipeptides and cyclopeptides were synthetized by solution phase peptide synthesis, using a Boc strategy. Diketopiperazine c(Val-Gly) (2) directly prepared starting from dipeptide Boc-Val-Gly-OMe (1). Deprotection Nα-Boc made by treating with TFA:DCM mixture 1:1 in an amount of 1ml for 100 mg of product. L'N-acetylation performed directly on DKP. Products (6a) and (6b), containing Δ^{z}Dmf and Δ^{z}Dmt, were prepared as shown in the following reaction: 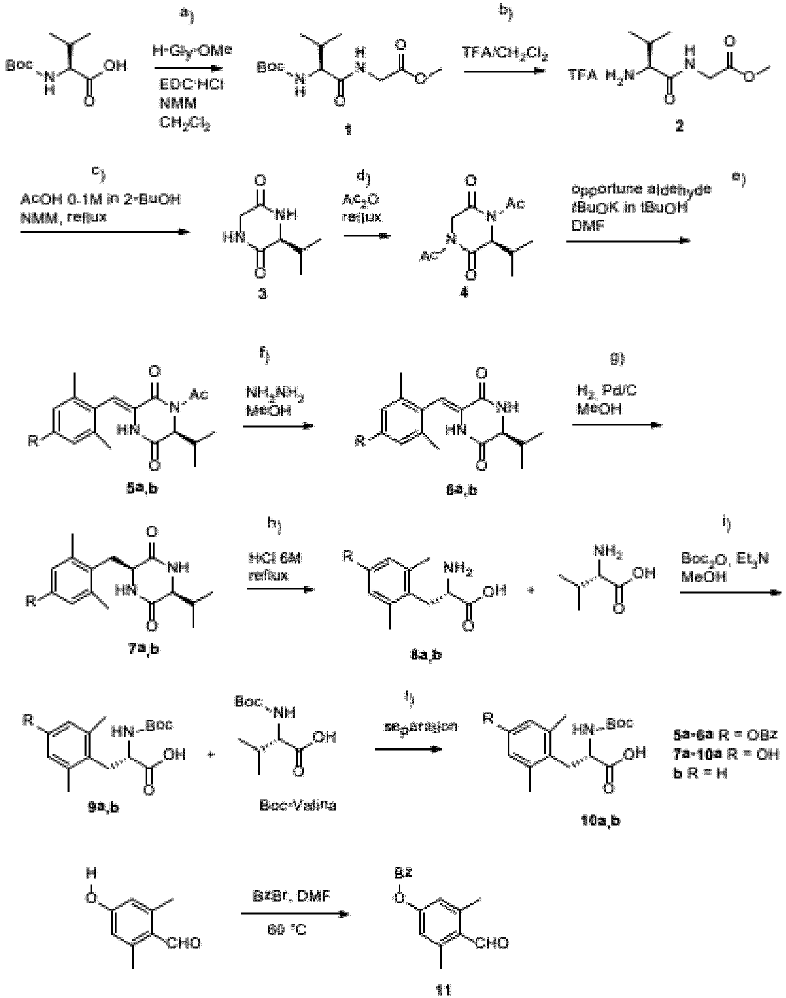 Conditions and reagents: a) Boc-Ala-OH, EDC·HCl, HOBt, NMM, DMF, t.a., overnight; b) TFA/CH₂Cl₂ 1:1, t.a., 1h, under N₂; c) AcOH/2-butanol, NMM, 120° C, 3h; d) Ac₂O boiling, overnight; e) 2',6'-dimethyl-PhCOH or 2',6'-dimethyl-*p*-OBz-Ph-CHO (**11**), tBuOK, DMF, t.a., 4h; f) NH₂-NH₂ monohydrate, MeOH, 2h; g) H₂, Pd/C-5% (10%p/p), 607950 Pa (6 atm); h) 6N HCl boiling, overnight; i) Boc₂O, NaHCO₃, Dioxane/H₂O; I) separation with HPLC , column C18, eluent water/acetonitrile; m) benzyl bromide, DMF, NaHCO₃.

### Product 1: Boc-Val-Gly-OMe

To an ice-cooled mixture Boc-Val-OH (1.1 eq.) in DMF were added EDC • HCl (1.1 eq.), HOBt (1.1 eq.), DIPEA (3.3 eq.) E HCl • H-Gly-OMe (1.0 eq.). The mixture heated at room temperature, stirred overnight and evaporated under reduced pressure. Residue dissolved in EtOAc and washed with three portions of citric acid 5%, NaHCO₃ saturated solution and NaCl saturated solution. Organic phases collected and dried on Na₂SO₄, and solvent evaporated under reduced pressure to give the desired product.
1H NMR (CDCl₃) δ: 6.65 (1H, t, Gly NH), 5.12 (1H, d, NHBoc), 4,32-4,25 (1H, m, Val αCH), 4,08-3,95 (2H, m, Gly CH₂), 3,75 (3H, s, OMe), 2.19 (1H, m, Val βCH), 1.35 (9H, s, Boc), 0,92-1,03 (6H, d, Val γCH3).

### Product 3: c(Val-Gly)

Boc-Val-Gly-OMe (product 1) was deprotected with a mixture of 50% TFA in CH₂Cl₂ at r.t. for 1h. The intermediate TFA salt (product 2) used for subsequent reactions without further purification. The methyl ester peptide, as trifluoroacetate salt (1 eq.) was dissolved in 0,1 M AcOH-2-butanol (1,5 eq.), and NMM added (1 eq.). The resulting weakly acid solution underwent to reflux in oil bath (120°C), overnight. The solvent was evaporated under vacuum, raw resuspeded in acetone and the product collected by filtration on paper, washed with small amounts of di cold H₂O and re-crystallized from a mixture of H₂O / 2-butanol to obtain the pure product.
¹H NMR (DMSO-d₆) δ: 7.45 (1H, s, NH), 7,38 (1H, s, NH), 4,60-4,55 (1H, d, Val αCH), 4,36-4,22 (2H, dd, Gly CH₂), 2.05 (1H, m, Val βCH), 0,95-1,10 (6H, d, Val γCH₃).

### Product 4: N, N'-diacetyl-c (Val-Gly)

A mixture of DKP (3) in acetic anhydride stirred under reflux for 12 hours. The solvent removed by azeotropic distillation with methanol and toluene under reduced pressure. The raw crystallized from EtOAc / Et₂O to obtain (4) as brown oil.
¹H NMR (CDCl₃) δ: 5,07-5,14 (1H, d, Gly CH₂), 5.01 (1H, d, Val αCH), 4,06-4,12 (1H, d, Gly CH₂), 2,60-2,57 (6H, s, 2 x N-Ac), 2.04 (1H, m, Val βCH), 1,11-0,97 (6H, d, Val γCH₃).

### Product 5b: c(Δ^{z}-DMP-N-acetil-Val)

Compound (4) (1 eq.) dissolved in DMF, then added 2,6-dimethylbenzaldehyde (1,5 eq.) and tBuOK 0,5M in tBuOH (1.5 eq.) drop by drop a 0°C. The mixture leaved to heat at r.t. and stirred for 7h. Then the reaction quenched with aqueous solution of NH₄Cl and extracted three times with EtOAc. The combined organic layers were dried on Na₂SO₄, filtered and evaporated. The raw purified by chromatography on silica gels using CH₂Cl₂/EtOAc 95:5 eluent to obtain the final product as colorless oil.
¹H NMR (CDCl₃) δ: 7,23-7,09 (4H, m, Ar, CH = C), 6,67 (1H, s, NH), 4,98 (1H, d, Val αCH), 2.63 (3H, s, N-ac), 2.20 (6H, s, Dmp CH₃), 2.08 (1H, m, Val βCH), 1,08-1,01 (6H, d, Val γCH₃).

### Compound 6b: c(Δ^{z}-DMP-Val)

The compound (5b) (1 eq.) was dissolved in MeOH and hydrazine monohydrate added. The product starts to precipitate from the solution. After 2 hthe solution was filtered and the pure final product was obtained as white dust in quantitative yield.
¹H NMR (DMSO-d₆) δ: 9.38 (1H, s, NH), 8.52 (1H, s, NH), 7,07-7,01 (3H, m, Ar), 6,62 (1H, s, CH = C), 3.79 (1H, m, Val αCH), 2.10 (6H, s, Dmp CH3), 2.02 (1H, m, Val βCH), 0,94-0,84 (6H, d, Val γCH₃).

### Compound 7b: c(DMP-Val)

The compound (**6b**) was dissolved in MeOH and H₂, Pd/C-5% (10%p/p), 607950 Pa (6 atm). Then the mixture hydrogenated for 24 hours in a Parr apparatus at 8 bar of H₂ a 45°C. The mixture was filtered to remove the catalyst and the solven evaporated to obtain the reduced product (**7b**) as white dust in quantitative yield.
¹H NMR (DMSO-d₆) δ: 1H NMR (DMSO-d6) δ: 8.28 (1H, d, NH), 7,93 (1H, d, NH), 6,99-6,98 (3H, m, Ar), 3,83 (1H, m, Val αCH), 3.51 (1H, m, Dmp αCH), 3,22-2,88 (2H, m, Dmp βCH₂), 2.25 (6H, s, Dmp CH₃), 2.03 (1H, m, Val βCH), 0,98-0,92 (6H, d, Val γCH₃).

### Product 10b: Boc-DMP-OH

DKP **7b,** was dissolved in HCl 12N (eq) and the solution stirred at 100°C for 24h. Then the solvent was evaporated under reduced pressure and the obtained dust was used for the following step without further purification. The mixture of HCl • H-DMP-OH (**8b**) and HCl • H-Val-OH was dissolved in dioxane/H₂O 1:1 (eq) 1 and NaHCO₃ (eq) and Boc₂O dissolved in dioxane added at 0 °C. the mixture heated at r.t. and stirred for 18h. Dioxane evaporated, pH adjusted at a 12 by adding NaOH 1N and the acqueous solution washed two times with Et₂O. Then the acqueous phase acidified at pH 2-3 by adding 2N HCl and three estraction with EtOAc were performed. The combined organic phase were dried on Na₂SO₄, filtered and evaporated under vacuum. The obtained raw of product **9b** and Boc-Val-OH, was puryfied by semi-preparative HPLC to obtain pure Boc-DMP-OH (**10b**) as white dust.
1H NMR (DMSO-d6)δ: 7.20 (1H, d, BocNH), 6,94-6,92 (3H, m, Ar), 3,43 (1H, m, αCH), 3,15-2,82 (2H, m, βCH₂), 2.25 (6H, s, 2 x CH₃), 1,35 e 1,29 (9H, s, Boc).

### Product 5a: c(O-Bn-Δ^{z}-Dmt-N-acetil-Val)

Compound (**5a**) was prepared from N,N'-diacetyl-c(Val-Gly) (**4**) and (**11**) following the same procedure of product (**5b**). After chromatographic purification the final product was obtained as colorless oil.
1H NMR (CDCl₃)δ: 7,45-7,33 (5H, m, OBn Ar), 7,12 (1H, s, CH = C), 7,05 (1H, s, NH), 5,06 (2H, s, CH₂-Ar), 4,97 (1H, d, Val αCH), 2.61 (3H, s, N-Ac), 2.17 (6H, s, Dmp CH₃), 2,07 (1H, m, Val βCH), 1,07-1,00 (6H, d , Val γCH₃).

### Product 6a: c(O-Bn-Δ^{z}-Dmt-Val)

Compound (**5a**) was deacetylated following the same procedure of product (**4b**). The precipitated was obtained as white dust in quantitative yield.
1H NMR (DMSO-d6) δ: 9.35 (1H, s, NH), 8.61 (1H, s, NH), 7,56-7,52 (5H, m, OBn Ar), 6,87 (2H, s, Ar), 6,67 (1H, s, CH = C), 5,26 (2H, s, CH₂-Ar), 3,76 (1H, m, Val αCH), 2.18 (6H, s, Dmt CH₃), 2,05 (1H, m, Val βCH), 0,95-0,88 (6H, d, Val γCH₃).

### Product 7a: c(Dmt-Val)

Hydrogenation of compound (**6a**) was performed following the same procedure of compound (**6b**) the reduced product was obtained as white dust in good yield.
1H NMR (DMSO-d6)δ: 1H NMR (DMSO-d6) δ: 9.01 (1H, s, Dmt OH), 8.23 (1H, d, NH), 7,80 (1H, d, NH), 6,39 (2H, s, Ar), 3.76 (1H, m, Val αCH), 3.50 (1H, m, Dmt αCH), 3,12-3,01 (2H, m, Dmt βCH₂), 2.16 (6H, s, Dmt CH₃), 2.04 (1H, m, Val βCH), 0,97-0,91 (6H, d, Val γCH₃).

### Product 10a: Boc-Dmt-OH

Hydrolysis and protection of the compound (**7a**) were performed with the same procedure of c(DMP-Val) (**7b**) to obtain a mixture composed by HCl · **8a** and HCl Val-OH. The mixture was Boc-protected in the same conditions of mixture HCl · **8b** and HCl · Val-OH and the so obtained Boc-protected product, consisting of product **9a** and Boc-Val-OH were separated by HPLC at the same conditions of products 9**b** and Boc-Val-OH. After preparative purification with HPLC pure Boc-DMT-OH (**10a**) was obtained as white dust.
1H NMR (CDCl₃)δ: 6.51 (2H, s, Ar), 4,79 (1H, d, BocNH), 4,96-4,42 (1H, m, αCH), 3,15-2,96 (2H, m, βCH₂), 2.31 (6H, s, 2 x CH₃), 1,45 e 1,37 (9H, s, Boc).

### Product 11: O-Bn-2,6-dimethylbenzaldehyde

2,6- dimethylbenzaldehyde (1 eq.) dissolved in DMF and benzyl bromide (eq.) added. The mixture stirred at 60°C and the reaction monitored by TLC. At the end, solvent was evaporated and the obtained product was used for the subsequent reaction without any purification.
1H NMR (CDCl₃)δ: 10.48 (1H, s, CHO), 7,42-7,40 (7H, m, Ar), 5,10 (2H, s, CH₂-Ar), 2,60 (6H, s, 2 x CH₃).

## Claims

1. Process for the stereo-selective preparation of L stereoisomers of unnatural aromatic amino acids optionally substituted on the aromatic ring of formula: 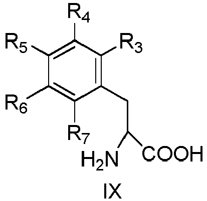 wherein the compound of formula (IX) is 2',6'-dimethyl-L-Tyrosine, 2',6'-dimethyl-L-Phenylalanine. Providing the following steps:
a) coupling amino acid L-valine having a protective group (GP) on the amine with a glycine methyl ester to obtain dipeptide of formula (I): 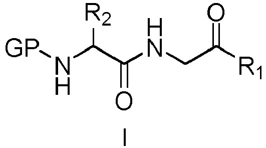 wherein R₁ is methoxy and R₂ is isopropyl
b) Removing the protective group (GP) from the compound of formula (I) as prepared in step a) by reacting with a suitable reactive to obtain dipeptide of formula (II) or salt thereof: 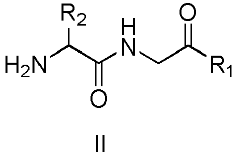 wherein R₁ is methoxy and R₂ is isopropyl
c) Intramolecular condensation of dipeptide of formula (II) as prepared in step b) to obtain compound of formula (III): 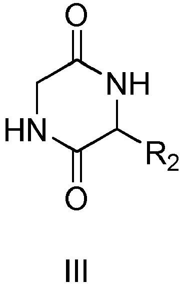 wherein R₂ is isopropyl
d) Acetylation of compound of formula (III) as prepared in step c) to obtain compound of formula (IV): 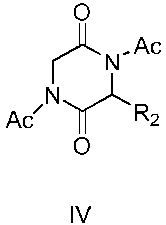 wherein R₂ is isopropyl and Ac is acetyl
e) Condensation of compound of formula (IV) as prepared in step d) with aldehyde of formula (V) to obtain Z-alkene of formula (VI): 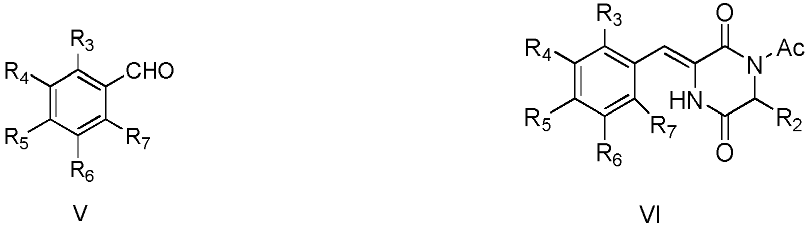
wherein R₂ is isopropyl
R3 and R7 are CH₃
R4 and R6 are H
R5 is benzyloxy or H
f) Removal of acetyl residue from compound of (VI) as prepared in step e) to obtain compound of formula (VII): 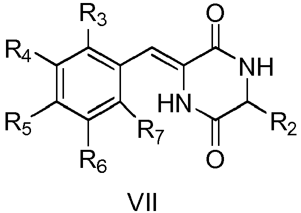
wherein R₂ is isopropyl
R3 and R7 are CH₃
R4 and R6 are H
R5 is benzyloxy or H
g) Catalytic hydrogenation of compound of formula (VII) as prepared in step f) in presence of a achiral catalyst, to obtain compound of formula (VIII): 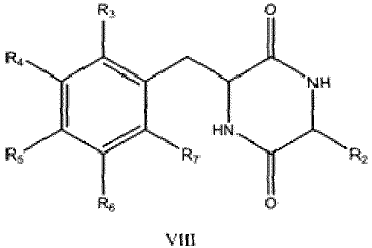
wherein R₂ is isopropyl
R3 and R7 are CH₃
R4 and R6 are H
R5 is H or OH
h) Hydrolysis of compound of formula (VIII) as prepared in step g) to obtain unnatural aromatic amino acids optionally substituted on the aromatic ring of formula (IX) in mixture with a amino acid of formula (X):
H2N-CH(R2)-COOH (X)
wherein R2 is isopropyl,

2. Process according to claim 1 further comprising the following steps:
i) protecting unnatural aromatic amino acids optionally substituted on the aromatic ring of formula (IX) and amino acids of formula (X) as prepared in step h) with a protector group GP on N-terminus, to obtain compounds of formula (XI) and (XII) 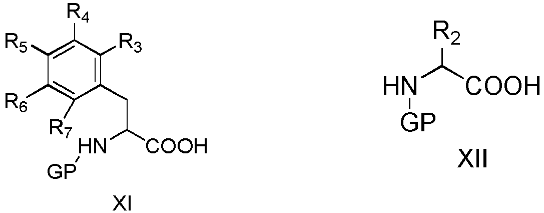
j) separation of compounds as prepared in step i) to obtain pure compound of formula (IX)
wherein
R2 is isopropyl,
R3 and R7 are CH₃
R4 and R6 are H
R5 is H or OH

3. Process according to claim 1 wherein in step b) salts of compound of formula (II) are selected from the group consisting of: salts of hydrochloric acid, salts of sulfuric acid, salts of trifluoroacetic acid, salts of hydrobromic acid.

4. Process according to claim 1 or 2 wherein in steps a) and i) the protective group (GP) is a tert-butyloxycarbonyl derivative (BOC).

5. Process according to claim 1 wherein in step b) the suitable reactant for eliminating the protective group is chosen in the group consisting of pure Trifluoroacetic acid, Trifluoroacetic acid in mixture with dichloromethane, chloridic acid in methanol or ethanol or THF or dioxane.

6. Process according to claim 1 wherein in step g) the achiral catalyst is 10% Palladium, 5% Palladium

7. Process according to claim 1 wherein the achiral catalyst is absorbed on coal.

8. Process according to claim 1 wherein in step g) the compound of formula (VII) is dissolved in MeOH before adding the catalyst.

9. Process according to claim 1 wherein in step g) hydrogenation is carried out for at least 24 hours, at hydrogen pressure between 202650 and 1013250 Pa (2 and 10 atmospheres) and temperature between 45 and 60 °C.

10. Process according to claim 1 wherein in step g) after hydrogenation mixture is filtered to remove catalyst and the solvent evaporated to obtain compound of formula (VIII).

## Patentansprüche

1. Verfahren zur stereoselektiven Herstellung von L-Stereoisomeren nicht-natürlicher aromatischer Aminosäuren, die optional am aromatischen Ring substituiert sind, der Formel: 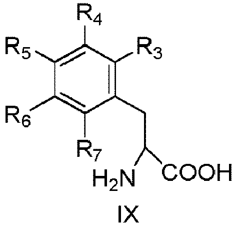 worin die Verbindung der Formel (IX) 2',6'-Dimethyl-L-tyrosin, 2',6'-Dimethyl-L-phenylalanin ist,
unter Bereitstellung der folgenden Schritte:
a) Verknüpfen der Aminosäure L-Valin, die eine Schutzgruppe (GP) am Amin trägt, mit einem Glycinmethylester, um das Dipeptid der Formel (I) zu erhalten: 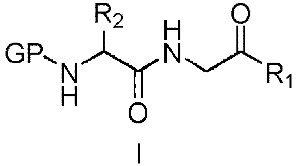 worin R₁ Methoxy ist und R₂ Isopropyl ist;
b) Entfernen der Schutzgruppe (GP) aus der Verbindung der Formel (I), wie sie in Schritt a) hergestellt wurde, durch Umsetzen mit einem geeigneten Reaktanten, um das Dipeptid der Formel (II) oder ein Salz davon zu erhalten: 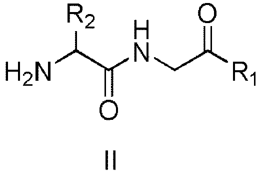 worin R₁ Methoxy ist und R₂ Isopropyl ist;
c) Intramolekulare Kondensation vom Dipeptid der Formel (II), wie es in Schritt b) hergestellt wurde, um die Verbindung der Formel (III) zu erhalten: 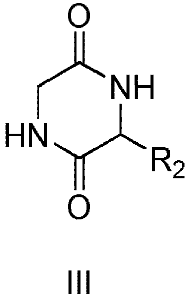 worin R₂ Isopropyl ist;
d) Acetylieren der Verbindung der Formel (III), wie sie in Schritt c) hergestellt wurde, um die Verbindung der Formel (IV) zu erhalten: 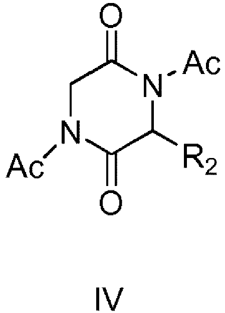 worin R₂ Isopropyl ist und Ac Acetyl ist
e) Kondensieren der Verbindung der Formel (IV), wie sie in Schritt d) hergestellt wurde, mit Aldehyd der Formel (V), um das Z-Alken der Formel (VI) zu erhalten: 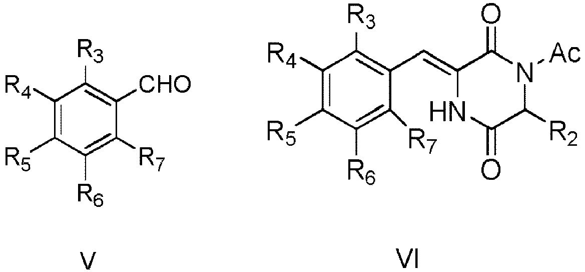
worin R₂ Isopropyl ist,
R3 und R7 CH₃ sind,
R4 und R6 H sind,
R5 Benzyloxy oder H ist;
f) Entfernen des Acetylrests der Verbindung nach (VI), wie sie in Schritt e) hergestellt wurde, um die Verbindung der Formel (VII) zu erhalten: 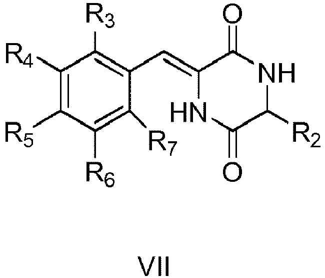
worin R₂ Isopropyl ist,
R3 und R7 CH₃ sind,
R4 und R6 H sind,
R5 Benzyloxy oder H ist;
g) Katalytische Hydrierung der Verbindung der Formel (VII), wie sie in Schritt f) hergestellt wurde, in Anwesenheit eines achiralen Katalysators, um die Verbindung der Formel (VIII) zu erhalten: 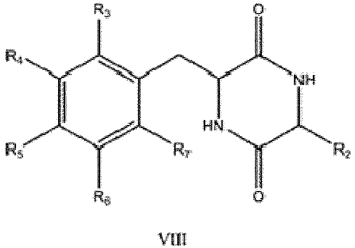
worin R₂ Isopropyl ist,
R3 und R7 CH₃ sind,
R4 und R6 H sind,
R5 H oder OH ist;
h) Hydrolyse der Verbindung der Formel (VIII), wie sie in Schritt g) hergestellt wurde, um eine nicht-natürliche aromatische Aminosäure, die optional am aromatischen Ring substituiert ist, der Formel (IX) in Mischung mit einer Aminosäure der Formel (X) zu erhalten:
H2N-CH(R2)-COOH (X)
worin R2 Isopropyl ist.

2. Verfahren, gemäß Anspruch 1, weiterhin die folgenden Schritte umfassend:
i) schützen nicht-natürlicher Aminosäuren, die optional am aromatischen Ring substituiert sind, der Formel (IX), und von Aminosäuren der Formel (X), wie sie in Schritt h) hergestellt wurden, mit einer Schutzgruppe GP am N-Terminus, um Verbindungen der Formel (XI) und (XII) zu erhalten. 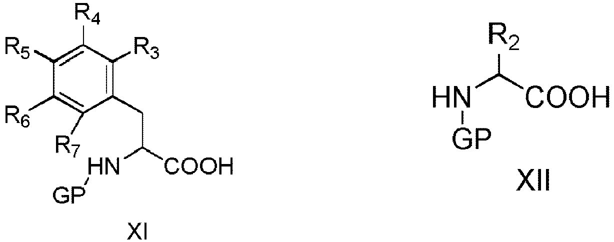
j) trennen der Verbindungen, wie sie in Schritt i) hergestellt wurden, um eine reine Verbindung der Formel (IX) zu erhalten,
worin
R2 Isopropyl ist,
R3 und R7 CH₃ sind,
R4 und R6 H sind,
R5 H oder OH ist.

3. Verfahren, gemäß Anspruch 1, worin in Schritt b) Salze von Verbindungen der Formel (II) ausgewählt sind aus der Gruppe, bestehend aus: Salzen von Salzsäure, Salzen von Schwefelsäure, Salzen von Trifluoressigsäure, Salzen von Bromwasserstoffsäure.

4. Verfahren, gemäß Anspruch 1 oder 2, worin in den Schritten a) und i) die Schutzgruppe (GP) ein tert-Butyloxycarbonylderivat (BOC) ist.

5. Verfahren, gemäß Anspruch 1, worin in Schritt b) der geeignete Reaktant zum Eliminieren der Schutzgruppe ausgewählt ist aus der Gruppe, die aus reiner Trifluoressigsäure, Trifluoressigsäure in Mischung mit Dichlormethan, Salzsäure in Methanol oder Ethanol oder THF oder Dioxan besteht.

6. Verfahren, gemäß Anspruch 1, worin in Schritt g) der achirale Katalysator 10% Palladium, 5% Palladium ist.

7. Verfahren, gemäß Anspruch 1, worin der achirale Katalysator auf Kohle absorbiert ist.

8. Verfahren, gemäß Anspruch 1, worin in Schritt g) die Verbindung der Formel (VII) in MeOH gelöst wird, bevor der Katalysator hinzugefügt wird.

9. Verfahren, gemäß Anspruch 1, worin in Schritt g) das Hydrieren mindestens 24 Stunden lang bei einem Wasserstoffdruck zwischen 202650 und 1013250 Pa (2 und 10 Atmosphären) und einer Temperatur zwischen 45 und 60 °C durchgeführt wird .

10. Verfahren, gemäß Anspruch 1, worin in Schritt g) nach dem Hydrieren die Mischung filtriert wird, um Katalysator zu entfernen, und das Lösemittel abgedampft wird, um die Verbindung der Formel (VIII) zu erhalten.

## Revendications

1. Procédé de préparation stéréosélective de stéréoisomères L d'acides aminés aromatiques non naturels facultativement substitués sur le cycle aromatique de formule : 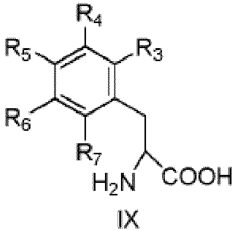 dans laquelle le composé de formule (IX) est la 2',6'-diméthyl-L-tyrosine, la 2',6'-diméthyl-L-phénylalanine.
Comprenant les étapes suivantes :
a) le couplage de l'acide aminé L-valine ayant un groupe protecteur (GP) sur l'amine avec un ester de méthyle de glycine pour obtenir un dipeptide de formule (I) : 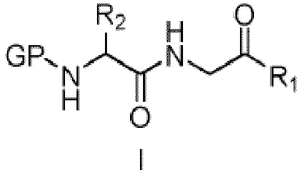 dans laquelle R₁ est méthoxy et R₂ est isopropyle
b) l'élimination du groupe protecteur (GP) à partir du composé de formule (I) tel que préparé dans l'étape a) par réaction avec un réactif approprié pour obtenir un dipeptide de formule (II) ou un sel de celui-ci : 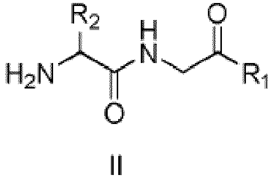 dans laquelle R₁ est méthoxy et R₂ est isopropyle
c) la condensation intramoléculaire du dipeptide de formule (II) tel que préparé dans l'étape b) pour obtenir un composé de formule (III) : 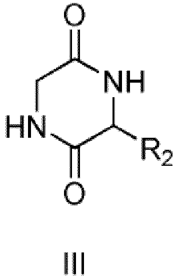 dans laquelle R₂ est isopropyle
d) l'acétylation du composé de formule (III) tel que préparé dans l'étape c) pour obtenir un composé de formule (IV) : 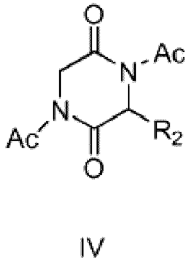 dans laquelle R₂ est isopropyle et Ac est acétyle
e) la condensation du composé de formule (IV) tel que préparé dans l'étape d) avec un aldéhyde de formule (V) pour obtenir un alcène Z de formule (VI) : 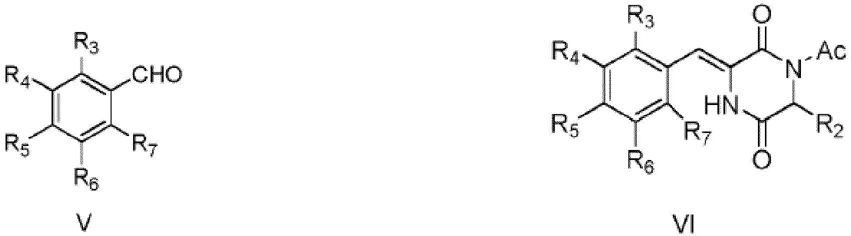
dans laquelle R₂ est isopropyle
R3 et R7 sont CH₃
R4 et R6 sont H
R5 est benzyloxy ou H
f) l'élimination du résidu acétyle à partir du composé de (VI) tel que préparé dans l'étape e) pour obtenir un composé de formule (VII) : 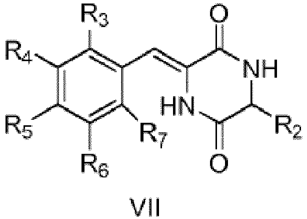
dans laquelle R₂ est isopropyle
R3 et R7 sont CH₃
R4 et R6 sont H
R5 est benzyloxy ou H
g) l'hydrogénation catalytique du composé de formule (VII) tel que préparé dans l'étape f) en présence d'un catalyseur achiral, pour obtenir un composé de formule (VIII) : 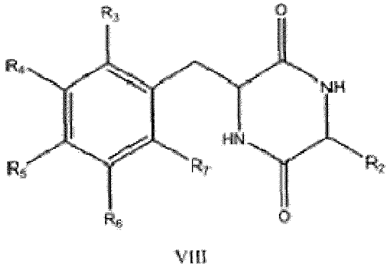
dans laquelle R₂ est isopropyle
R3 et R7 sont CH₃
R4 et R6 sont H
R5 est H ou OH
h) l'hydrolyse du composé de formule (VIII) tel que préparé dans l'étape g) pour obtenir des acides aminés aromatiques non naturels facultativement substitués sur le cycle aromatique de formule (IX) en mélange avec un acide aminé de formule (X) :
H2N-CH(R2)-COOH (X)
dans lequel R₂ est isopropyle.

2. Procédé selon la revendication 1 comprenant en outre les étapes suivantes :
i) la protection des acides aminés aromatiques non naturels facultativement substitués sur le cycle aromatique de formule (IX) et des acides aminés de formule (X) tels que préparés dans l'étape h) avec un groupe protecteur GP à l'extrémité N-terminale, pour obtenir des composés de formules (XI) et (XII) 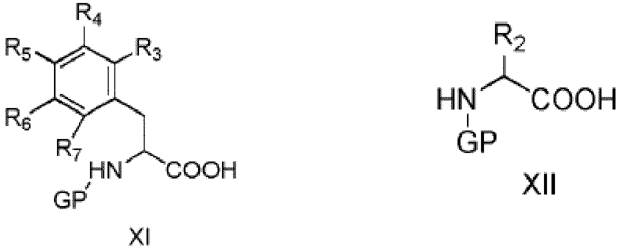
j) la séparation des composés tels que préparés dans l'étape i) pour obtenir un composé pur de formule (IX)
dans laquelle
R2 est isopropyle
R3 et R7 sont CH₃
R4 et R6 sont H
R5 est H ou OH.

3. Procédé selon la revendication 1 dans lequel dans l'étape b) des sels du composé de formule (II) sont sélectionnés dans le groupe constitué : des sels d'acide chlorhydrique, des sels d'acide sulfurique, des sels d'acide trifluoroacétique, des sels d'acide bromhydrique.

4. Procédé selon la revendication 1 ou 2 dans lequel dans les étapes a) et i) le groupe protecteur (GP) est un dérivé de tert-butyloxycarbonyle (BOC).

5. Procédé selon la revendication 1 dans lequel dans l'étape b) le réactif approprié pour éliminer le groupe protecteur est choisi dans le groupe constitué de l'acide trifluoroacétique pur, de l'acide trifluoroacétique en mélange avec le dichloro-méthane, de l'acide chlorhydrique dans le méthanol ou l'éthanol ou le THF ou le dioxane.

6. Procédé selon la revendication 1 dans lequel dans l'étape g) le catalyseur achiral est du palladium à 10 %, du palladium à 5 %.

7. Procédé selon la revendication 1 dans lequel le catalyseur achiral est absorbé sur du charbon.

8. Procédé selon la revendication 1 dans lequel dans l'étape g) le composé de formule (VII) est dissous dans du MeOH avant l'ajout du catalyseur.

9. Procédé selon la revendication 1 dans lequel dans l'étape g) l'hydrogénation est réalisée pendant au moins 24 heures, à une pression d'hydrogène comprise entre 202 650 et 1 013 250 Pa (2 et 10 atmosphères) et à une température comprise entre 45 et 60 °C.

10. Procédé selon la revendication 1 dans lequel dans l'étape g) après l'hydrogénation le mélange est filtré pour éliminer le catalyseur et le solvant est évaporé pour obtenir un composé de formule (VIII).
